# EUROPÄISCHE PATENTANMELDUNG

(11) **EP 1 103 220 A2**
(43) Veröffentlichungstag der Anmeldung: **30.05.2001**
(21) Anmeldenummer: 00204060.8
(22) Anmeldetag: 16.11.2000
(51) Int. Cl.: A61B 6/03

(54) **Computertomographie-Verfahren**

(30) Priorität: 25.11.1999 DE 19956585
(71) Anmelder: Philips Corporate Intellectual Property GmbH, 52064 Aachen (DE); Koninklijke Philips Electronics N.V., 5621 BA Eindhoven (NL)
(72) Erfinder: Köhler, Thomas, Dr., 52064 Aachen (DE)
(74) Vertreter: Volmer, Georg, Dipl.-Ing.

(57) **Zusammenfassung**

Die Erfindung betrifft ein Computertomographie-Verfahren, bei dem ein verbesserter Kompromiß zwischen Übertragungsbandbreite und Bildqualität durch folgende Schritte erzielt wird:
- Kombination der Signale mindestens je zweier benachbarter Detektorelemente zu je einem Meßwert
- zyklische Variation der Kombinationen von Strahlenquellenposition zu Strahlenquellenposition.

## Beschreibung

Die Erfindung betrifft ein Computertomographie-Verfahren mit den Schritten
- Erzeugen eines einen Untersuchungsbereich bzw. ein darin befindliches Objekt durchsetzenden Strahlenbündels mit einer Strahlenquelle,
- Erzeugung einer eine Rotation um eine Rotationsachse umfassenden Relativbewegung zwischen der Strahlenquelle einerseits und dem Untersuchungsbereich bzw. dem Objekt andrerseits,
- Akquisition von Meßwerten für eine Vielzahl von Strahlenquellenpositionen mit einer mit der Strahlenquelle gekoppelten Detektoreinheit, die mindestens eine Zeile von Detektorelementen umfaßt.
Außerdem bezieht sich die Erfindung auf einen Computertomographen zur Durchführung des Verfahrens.

Die Zahl der Auslesekanäle bei einem Computertomographen ist mit Rücksicht auf die Kosten für die Datenakquisition, die Übertragungsbandbreite des Datenschleifrings und/oder den Datendurchsatz begrenzt, den eine Rekonstruktionseinheit verarbeiten kann. Beispielsweise müssen bei einem Computertomographen, der pro Sekunde dreimal um einen Untersuchungsbereich rotiert und bei jeder Umdrehung aus 1.400 verschiedenen Winkelstellungen Daten mit einer Detektoreinheit akquiriert, die 16 Zeilen mit je 1.000 Detektorelementen umfaßt, die Meßwerte mit einer Übertragungsbandbreite von rund 200 MByte/Sekunde übertragen und ausgewertet werden (unter der Annahme das ein Meßwert mit 3 Byte übertragen wird). Dies wäre - wenn überhaupt - nur mit einem erheblichen Aufwand möglich.

Aus der GB-PS 2,005,955 ist bereits ein Computertomograph der eingangs genannten Art bekannt, bei dem die Signale von zwei oder mehr Detektorelementen am Rande der Detektoreinheit kombiniert werden. Auf diese Weise müssen weniger Meßwerte zu der Rekonstruktionseinheit, die aus den Meßwerten Computertomogramme rekonstruiert, übertragen werden; jedoch wird dies mit einer Verringerung der räumlichen Auflösung zumindest an den Rändern des Untersuchungsbereichs erkauft.

Eine solche Kombination von Signalen würde bei Computertomographen mit der heute üblichen sogenannten "Quarter-Detector-Shift" (dabei ist die Detektoreinheit in bezug auf die Strahlenquelle und die Rotationsachse so angeordnet, dass die Projektionen der Rotationsachse auf die Detektoreinheit um ein Viertel der Breite eines Detektorelements gegenüber der Mitte der Detektoreinheit versetzt ist) zu einer weiteren Verringerung des Auflösungsvermögens führen, weil die mit der Quarter-Detector-Shift verbundenen Vorteile nicht mehr ausgenutzt werden können.

Aufgabe der vorliegenden Erfindung ist es, ein Computertomographie-Verfahren der eingangs genannten Art so auszugestalten, dass sich ein günstigerer Kompromiß zwischen der Zahl der zu messenden/zu übertragenden und/oder zu verarbeitenden Meßwerte einerseits und der Bildqualität andererseits ergibt.

Diese Aufgabe wird ausgehend von einem Computertomographie-Verfahren der eingangs genannten Art erfindungsgemäß gelöst durch folgende Schritte:
- Kombination der Signale mindestens je zweier benachbarter Detektorelemente zu je einem Meßwert
- zyklische Variation der Kombinationen von Strahlenquellenposition zu Strahlenquellenposition.

Die Erfindung basiert auf folgenden Überlegungen.

In einer Detektoreinheit hat jedes Detektorelement verschiedene Nachbar-Detektor-elemente. Man kann also die Ausgangssignale der Detektorelemente auf verschiedene Weise miteinander kombinieren. Jede Kombination für sich allein würde, wie zuvor erläutert, zu einer Verringerung des Auflösungsvermögens führen. Wenn jedoch beim Übergang von einer Strahlenquellenpostion zur nächsten von einer der erwähnten Kombinationen dynamisch auf eine andere umgeschaltet wird, so dass die Kombinationen zyklisch durchlaufen werden, dann ergibt sich nur ein geringer Verlust an räumlichen Auflösungsvermögen. Dies liegt daran, dass sich die Kombinationen so ergänzen, dass die Meßwerte sich im Radon-Raum nahezu in derselben Weise wie ohne diese Signalkombinationen verteilen.

Ein Computertomograph zur Durchführung des erfindungsgemäßen Verfahrens ist in Anspruch 2 angegeben. Gemäß der Ausgestaltung nach Anspruch 3 handelt es sich dabei vorzugsweise um einen solchen mit kegelförmigen Strahlenbündel (,,cone beam CT"). Zwar ergeben sich die Vorteile der Erfindung auch dann, wenn die Detektoreinheit nur eine Zeile aufweist ("fan beam CT"), jedoch ist die Erfindung bei cone beam CT nötiger als bei fan beam CT.

Eine erste Möglichkeit zur Kombination von Detektorelementen ist in Anspruch 4 angegeben. Diese Kombination der Signale aus benachbarten Spalten (aber derselben Zeile) ist gemäß Anspruch 5 vorzugsweise in Verbindung mit dem sogenannten "Quarter Detector Shift" anwendbar. Gemäß Anspruch 6 können aber auch die Signale von Detektor-Elementen aus benachbarten Zeilen miteinander kombiniert werden. Die Ausgestaltungen nach den Ansprüchen 4 bis 6 sind aber auch gemeinsam anwendbar.

Die Erfindung wird nachstehend anhand der Zeichnungen näher erläutert. Es zeigen:
Fig. 1 einen Computertomographen, mit dem die Erfindung ausführbar ist,
Fig. 2a) bis 2c) eine Detektoreinheit und dabei mögliche Kombinationen von Detektorelementen,
Fig. 3 die zeitliche Abfolge der verschiedenen Kombinationen in aufeinander folgenden Strahlenquellenpositionen,
Fig. 4a) bis 4d) die resultierende Abtastung des Radon-Raums,
Fig. 5a) bis 5d) die bei einer zweiten Ausführungsform resultierende Abtastung des Radon-Raums,
Fig. 6a) bis 6c) die bei einer dritten Ausführungsform möglichen Kombinationen und
Fig. 7 eine Kombinationseinheit zur Kombination der Signale verschiedener Detektorelemente in schematischer Darstellung.

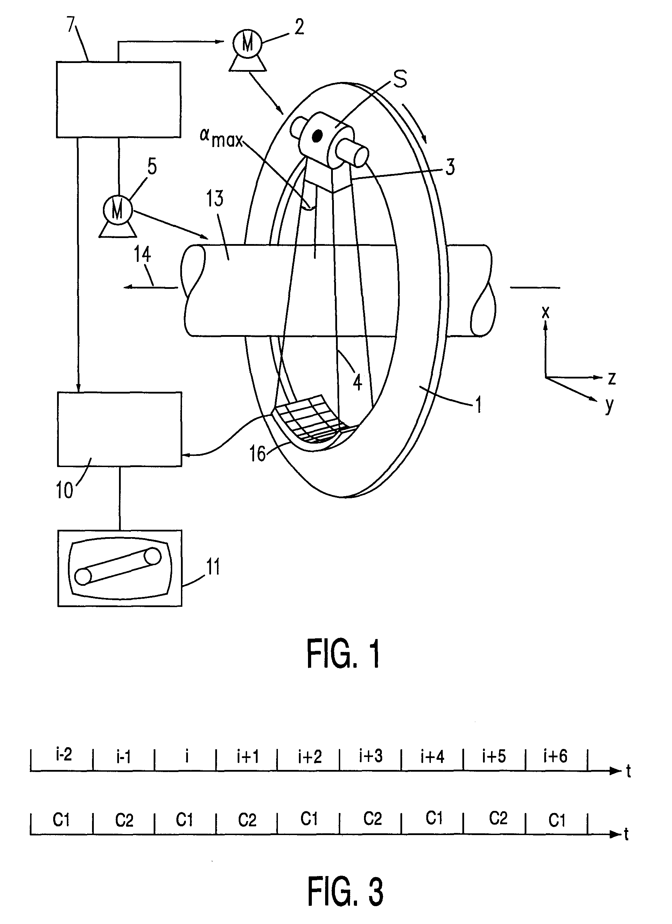

Der in Fig. 1 dargestellte Computertomograph umfaßt eine Gantry 1, die um eine Rotationsachse 14 rotieren kann. Dazu wird die Gantry von einem Motor 2 mit einer vorzugsweise konstanten, aber einstellbaren Winkelgeschwindigkeit angetrieben. An der Gantry 1 ist eine Strahlenquelle S, beispielsweise ein Röntgenstrahler, befestigt. Dieser ist mit einer Kollimatoranordnung 3 versehen, die aus der von der Strahlenquelle S erzeugten Strahlung ein kegelförmiges Strahlenbündel 4 ausblendet, d.h. ein Strahlenbündel, das sowohl in einer zur Rotationsachse senkrechten Ebene als auch in Richtung der Rotationsachse eine von Null verschiedene endliche Ausdehnung hat.

Das Strahlenbündel 4 durchdringt einen Untersuchungsbereich 13, in dem sich ein Patient auf einen Patientenlagerungstisch (beides nicht näher dargestellt) befinden kann. Der Untersuchungsbereich 13 hat die Form eines Zylinders. Nach dem Durchsetzen dieses Zylinders trifft das Röntgenstrahlenbündel 4 auf einer an der Gantry 1 befestigte zweidimensionale Detektoreinheit 16, die eine Vielzahl von matrixförmig angeordneten Detektorelementen umfaßt. Jedes Detektorelement kann in jeder Strahlenquellenposition einen Meßwert für einen Strahl aus dem Strahlenbündel 4 liefern. Die Detektorelemente sind in Zeilen und Spalten angeordnet. Die Detektorzeilen befinden sich in zur Rotationsachse senkrechten Ebenen, beispielsweise auf einem Kreisbogen um die Strahlenquelle S. Die Detektorspalten verlaufen parallel zur Rotationsachse. Die Detektorzeilen enthalten in der Regel wesentlich mehr Detektorelemente (z.B. 1.000) als die Detektorspalten (z.B. 16).

Der mit αₘₐₓ bezeichnete Öffnungswinkel des Strahlenbündels 4 (als Öffnungswinkel ist der Winkel definiert, den ein Strahl, der in einer zur Rotationsachse 14 senkrechten Ebene am Rande des Strahlenbündels 4 liegt, mit einer durch die Strahlenquelle S und die Rotationsachse definierten Ebene einschließt) bestimmt dabei den Durchmesser des Zylinders, innerhalb dessen sich das zu untersuchende Objekt bei der Äquisition der Meßwerte befinden muß. Das Untersuchungsobjekt bzw. der Patientenlagerungstisch (beides nicht dargestellt) kann mittels eines Motors 5 auch parallel zur Rotationsachse 14 verschoben werden. Die Geschwindigkeit dieses Vorschubs ist vorzugsweise konstant und einstellbar. Wenn die Motoren 5 und 2 gleichzeitig laufen, ergibt sich eine helixförmige Abtastbewegung der Strahlenquelle S und der Detektoreinheit 16. Wenn hingegen der Motor 5 für den Vorschub in Richtung der Rotationsachse still steht und der Motor 2 die Gantry rotieren läßt, ergibt sich eine kreisförmige Abtastbewegung der Strahlenquelle S und der Detektoreinheit relativ zum Untersuchungsbereich. Die Erfindung ist bei beiden Abtastbewegungen anwendbar.

Die von der Detektoreinheit 16 auf der rotierenden Gantry 1 akquirierten Meßdaten werden - nachdem sie auf die erfindungsgemäße Weise kombiniert worden sind - einem Bildverarbeitungsrechner 10 zugeführt, der sich in der Regel an einem festen Punkt im Raum befindet und mit der Detektoreinheit über einen kontaktlos arbeitenden nicht näher dargestellten Datenschleifring verbunden ist.

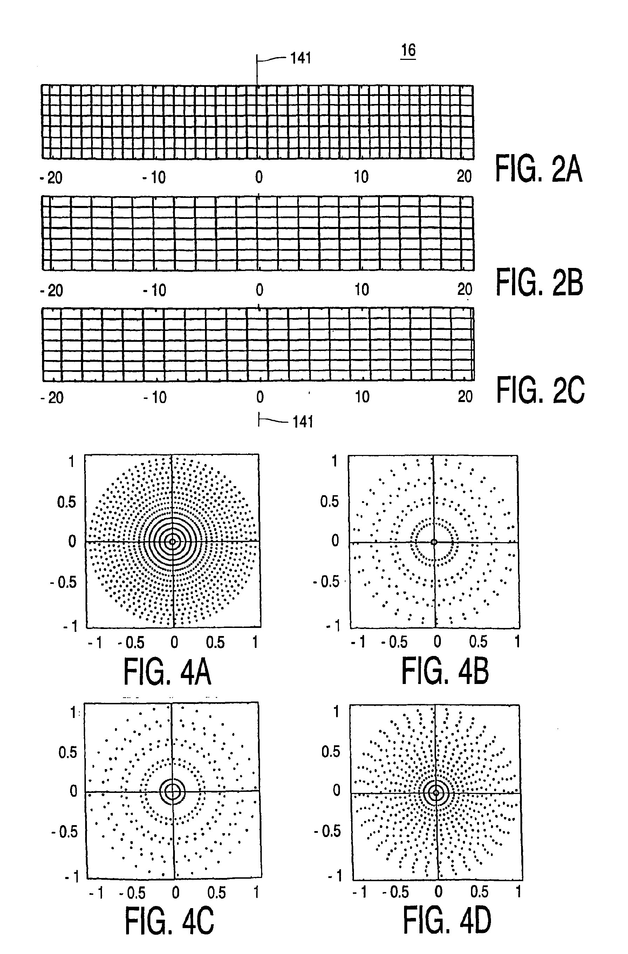

Fig. 2a) zeigt eine Abwicklung der Detektoreinheit 16 in vereinfachter Darstellung, d.h. mit einer verringerten Anzahl von Zeilen und Spalten von Detektorelementen, die jeweils durch ein Quadrat angedeutet sind. Die Spalten sind von der Mitte ausgehend nach außen positiv bzw. negativ numeriert. Mit 141 ist dabei die Gerade bezeichnet, die sich bei einer Projektion der Rotationsachse 14 durch die Strahlenquelle S auf die Detektoreinheit ergibt. Diese Gerade ist zugleich die Schnittgerade der Detektoreinheit 16 mit einer Ebene, die durch die Strahlenquelle und die Rotationsachse definiert ist. Man erkennt, dass die Mitte der Detektoreinheit (die durch den Außenrand der beiden mittleren Detektorelemente in einer Detektorzeile definiert ist) gegenüber der Geraden 141 um eine Strecke versetzt ist, die einem Viertel der Breite eines Detektorelements entspricht (Quarter-Detector Shift).

Fig. 4a) zeigt die Abtastung des Radon-Raumes durch die mittlere Detektorzeile, wobei angenommen ist, dass diese Detektorzeile lediglich 20 Detektorelemente enthält und dass der Untersuchungsbereich aus 72 gleichmäßig verteilten (d.h. um 5° bezüglich der Rotationsachse gegeneinander versetzten) Strahlenquellenpositionen durchstrahlt worden ist. Jeder Punkt stellt einen Meßwert eines Detektorelements der Zeile für eine der Strahlenquellenpositionen dar. Dieser Punkt befindet sich jeweils am Fußpunkt eines Lotes vom Zentrum (0,0) des Radon-Raumes auf den Strahl dar, der die Strahlenquelle in der betreffenden Strahlenquellenposition mit dem erwähnten Detektorelement verbindet. Das Zentrum (0,0) ist dabei der Schnittpunkt der betrachteten Ebene mit der Rotationsachse. Ihm ist der betreffende Meßwert zugeordnet, der dem Linienintegral der Absorption entlang des Strahls entspricht. Je dichter die Punkte im Radon-Raum in radialer Richtung liegen, desto größer ist (bei gleichmäßiger Abtastung des Radon-Raums) die räumliche Auflösung, die mit einer Rekonstruktion erzielbar sind.

Erfindungsgemäß werden nun die Ausgangssignale von je zwei Detektorelementen aus derselben Zeile, aber aus benachbarten Spalten, miteinander kombiniert. Dafür gibt es zwei mögliche Kombinationen. Bei der ersten Kombination werden die Ausgangssignale jedes zweiten Detektorelements der Zeile mit den Ausgangssignalen des jeweils rechten Nachbardetektorelements kombiniert. Die zweite Kombinationsmöglichkeit besteht darin, die Ausgangssignale dieser Detektorelemente und ihrer jeweils links liegenden Nachbardetektorelemente zu kombinieren. Die resultierende Verschiebung des Detektors gegenüber der Geraden 141 beträgt 1/8 bzw 3/8, wobei zu beachten ist, dass eine 3/8-Verschiebung nach links identisch ist mit einer 5/8-Verschiebung nach rechts.

Diese Kombinationsmöglichkeiten sind in den Fig. 2b) und 2c) schematisch angedeutet, wobei im Vergleich zu Fig. 2a) die Detektorelemente, deren Ausgangssignale kombiniert werden, durch ein gemeinsames Rechteck symbolisiert sind. Man erkennt, dass die Rechtecke in Fig. 2b) gegenüber denen von Fig. 2c) um die Breite eines Detektorelements (in Fig. 2a)) gegeneinander versetzt sind.

Fig. 3c) zeigt den zeitlichen Ablauf während der Datenakquisition. Die erste Zeile stellt die jeweilige Strahlenquellenposition i-2, i-1, i,i+1, ... i+6 als Funktion der Zeit dar. Die zweite Zeile gibt an, welche der beiden Kombinationsmöglichkeiten gewählt sind, wobei c1 beispielsweise für die Kombination gemäß Fig. 2b) steht und c2 für die Kombination nach Fig. 2c). Man erkennt, dass von Strahlenquellenposition zu Strahlenquellenposition zyklisch zwischen diesen beiden Kombinationen umgeschaltet wird.

Fig. 4b) zeigt die Abtastung des Radon-Raums durch die Anordnung nach Fig. 2b). Die Zahl der Strahlenquellenpositionen (36) ist - im Vergleich zu Fig. 4a - genauso halbiert wie die Zahl der Meßwerte pro Strahlenquellenposition (10). Man erkennt, dass der Radon-Raum dabei in radialer Richtung ungleichmäßig abgetastet wird, was zu einer Verringerung des räumlichen Auflösungsvermögens führt.

Fig. 4c) zeigt das entsprechende Diagramm im Radon-Raum für die dazwischen liegenden 36 Strahlenquellenpositionen mit der Anordnung nach Fig. 2c).

Fig. 4d) zeigt schließlich die Abtsatung des Radon-Raumes, die resultiert, wenn man gemäß Fig. 3 fortlaufend zwischen den beiden Kombinationen hin und her schaltet. Man erkennt, dass der Radon-Raum in radialer Richtung praktisch gleich gut abgetastet wird wie bei Fig. 4a), obwohl infolge der Zusammenfassung von Meßwerten nur die Hälfte der Meßwerte erzeugt wird.

Die Kombination von Ausgangssignalen benachbarter Detektorelemente hat, wie bereits erwähnt, denselben Effekt, als wenn entsprechend breitere Detektorelemente verwendet würden. Infolgedessen sind die Strahlen von der Strahlenquelle bis zum Detektorelement breiter, wodurch die Auflösung sich - im Vergleich zu der Detektoreinheit nach Fig 2a - verschlechtert, da über die Fläche der Detektorelemente gemittelt wird. Hier läßt sich eine Verbesserung durch zusätzliche Entfaltungstechniken erreichen, wie sie z.B. auch bei der Korrektur von Detektor Cross-Talk verwendet werden.

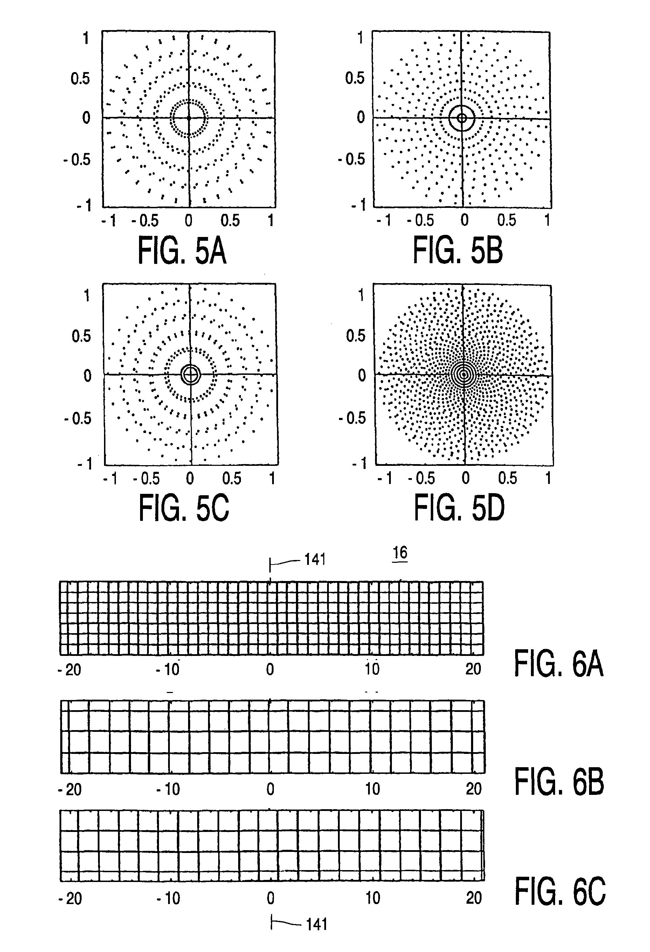

Man kann auch die Ausgangssignale von N (N>2) benachbarten Detektorelementen miteinander kombinieren. In diesem Fall ergibt sich eine optimale Abtastung, wenn N verschiedene Kombinationen mit einer Verschiebung der aus N Detektorelementen bestehenden Gruppe um 1/4N, 3/4N ... (2N-1)/4N der Breite eines Detektorelements benutzt. In den Fig. 5a) ... 5d) ist die resultierende Abtastung des Radon-Raumes für N=3 dargestellt. Dabei ist angenommen, dass durch die Kombination der Ausgangssignale von je drei benachbarten Detektorelementen pro Strahlenquellenposition zehn Meßwerte resultieren und dass für jedes Diagramm 36 Messungen aus gleichmäßig (jeweils um 10°) versetzten Strahlenquellenpositionen erfolgen, wobei die 36 Strahlenquellenpositionen des einen Diagramms gegenüber denen des anderen um jeweils 3,33° versetzt sind.

Fig. 5a) zeigt das Diagramm für einen Detektorversatz von 1/12 Detektorbreite, Fig. 5b) für 3/12 und Fig. 5c) für 5/12 Detektorbreire, Fig. 5d) zeigt die Abtastung des Radon-Raums, wenn man von Strahlenquellenposition zu Strahlenquellenposition zyklisch zwischen diesen drei Kombinationsmöglichkeiten umschaltet. Obwohl durch die Kombination jeweils nur noch 1/3 der Meßwerte übertragen werden, ist die Abtastung des Radon-Raums in radialer Richtung gleich gut als wenn die Ausgangssignale der Detektorelemente nicht miteinander kombiniert werden würden.

Bei den vorstehend erläuterten Ausführungsbeispielen wurden jeweils die Ausgangssignale von Detektorelementen aus der gleichen Zeile und zwei oder mehr benachbarten Spalten zusammengefaßt. Ebenso ergibt sich eine Verringerung der Anzahl der zu übertragenden Signale mit einer wesentlich geringeren Verschlechterung des räumlichen Auflösungsvermögens, wenn man die Ausgangssignale von Detektorelementen aus derselben Spalte, aber aus benachbarten Zeilen miteinander kombiniert. Man kann aber auch die Ausgangssignale von benachbarten Zeilen und von benachbarten Spalten miteinander kombinieren. Dies wird im folgenden anhand der Fig. 6a), 6b) und 6c) erläutert.

Fig. 6a) zeigt wiederum die Detektoreinheit 16, wobei ein Quadrat jeweils ein Detektorelement darstellt. Faßt man die Ausgangssignale von je zwei benachbarten Zeilen und Spalten zusammen und stellt man die Fläche der auf diese Weise zusammengeschalteten Detektorelemente durch ein Quadrat dar, dann geht die Anordnung nach Fig. 6a) in Fig. 6b) über oder in die nach Fig. 6c). Die Fig. 6b) und Fig. 6c) unterscheiden sich dadurch voneinander, dass sowohl die Zeilen als auch die Spalten, deren Detektorelemente miteinander kombiniert werden, vertauscht werden. Die resultierenden Quadrate sind dabei jeweils um die Abmessungen eines Detektorelements nach Fig. 6a) in Zeilen- und in Spaltenrichtung gegeneinander verschoben. Die Zahl der zu übertragenden Meßwerte wird hierbei auf 1/4 reduziert, ohne dass das räumliche Auflösungsvermögen in gleicher Weise abnimmt.

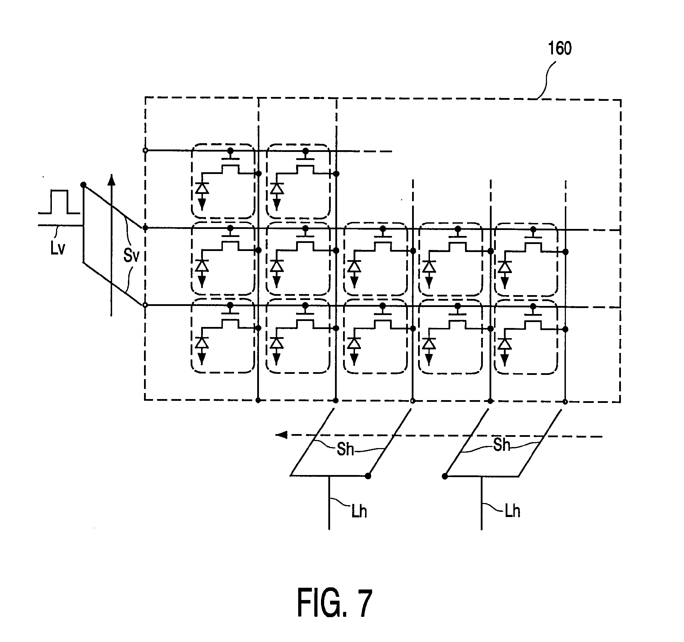

In Fig. 7 ist schematisch ein Ausschnitt 160 einer Detektoreinheit angedeutet, die eine Anzahl von matrixförmigen Detektorelementen umfaßt. Der Meßwert eines Detektorelements läßt sich auslesen, wenn die zu diesem Detektorelement gehörende horizontal verlaufende Schaltleitung aktiviert ist und wenn das Signal auf der mit diesem Detektorelement verbundenen Ausleseleitung verarbeitet wird. Um die Ausgangssignale von je zwei in horizontaler Richtung und je zwei in vertikaler Richtung zueinander benachbarten Detektorelementen zusammenfassen zu können, sind je zwei Schaltleitungen über ein elektronisches Schalterpaar S_{V} in einer ersten Schalterstellung mit einer Schaltleitung L_{V} verbindbar. Ebenso sind je zwei Ausleseleitungen über ein Schalterpaar in einem ersten Betriebsmodus mit einer gemeinsamen Ausleseleitung L_{H} verbindbar, wobei die Ausleseleitungen mit nicht näher dargestellten elektronischen Verarbeitungsmitteln (z.B. Ausleseverstärkern, Multiplexern usw.) verbunden sind. In einem zweiten Betriebsmodus werden die Schalter in Pfeilrichtung verstellt, so dass jeweils zwei andere benachbarte Schaltleitungen der Detektoreinheit 160 mit der gemeinsamen Schaltleitung L_{V} und jeweils zwei andere Ausleseleitungen mit der gemeinsamen Ausleseleitung L_{H} verbunden werden.

## Patentansprüche

1. Computertomographie-Verfahren mit den Schritten
• Erzeugen eines einen Untersuchungsbereich bzw. ein darin befindliches Objekt durchsetzenden Strahlenbündels mit einer Strahlenquelle,
• Erzeugung einer eine Rotation um eine Rotationsachse umfassenden Relativbewegung zwischen der Strahlenquelle einerseits und dem Untersuchungsbereich bzw. dem Objekt andererseits,
• Akquisition von Meßwerten für eine Vielzahl von Strahlenquellenpositionen mit einer mit der Strahlenquelle gekoppelten Detektoreinheit, die mindestens eine Zeile von Detektorelementen umfaßt,
gekennzeichnet durch folgende Schritte:
• Kombination der Signale mindestens je zweier benachbarter Detektorelemente zu je einem Meßwert
• zyklische Variation der Kombinationen von Strahlenquellenposition zu Strahlenquellenposition.

2. Computertomograph zur Durchführung des Verfahrens nach Anspruch 1 mit
• einer Strahlenquelle zum Erzeugen eines einen Untersuchungsbereich bzw. ein darin befindliches Objekt durchsetzenden Strahlenbündels,
• einer Antriebseinheit für eine Rotation um eine Rotationsachse umfassende Relativbewegung zwischen der Strahlenquelle einerseits und dem Untersuchungsbereich bzw. dem Objekt andererseits,
• einer mit der Strahlenquelle gekoppelten Detektoreinheit, die mindestens eine Zeile von Detektorelementen umfaßt, zur Akquisition von Meßwerten für eine Vielzahl von Strahlenquellenpositionen
gekennzeichnet durch
• eine Kombinationseinrichtung zur Kombination der Signale von mindestens je zweier benachbarten Detektorelemente zu je einem Meßwert
• Mittel zur zyklischen Variation der Kombinationen von Strahlenquellenposition zu Strahlenquellenposition.

3. Computertomograph nach Anspruch 2
dadurch gekennzeichnet,
• dass die Strahlenquelle eine Blendenvorrichtung aufweist zur Ausblendung eines kegelförmigen Strahlenbündels und
• dass die Detektoreinheit mehrere Zeilen von Detektorelementen umfaßt.

4. Computertomograph nach Anspruch 2
dadurch gekennzeichnet,
dass die Kombinationseinrichtung so ausgebildet ist, dass die Signale von Detektorelementen aus benachbarten Spalten der Detektoreinheit miteinander kombiniert werden.

5. Computertomograph nach Anspruch 4
dadurch gekennzeichnet,
dass die Detektoreinheit in Bezug auf die Strahlenquelle und die Rotationsachse so angeordnet ist, dass die Projektion der Rotationsachse auf die Detektoreinheit um ein Viertel der Breite eines Detektorelements gegenüber der Mitte der Detektoreinheit versetzt ist.

6. Computertomograph nach Anspruch 2
dadurch gekennzeichnet,
dass die Kombinationseinrichtung so ausgebildet ist, dass die Signale von Detektorelementen aus benachbarten Zeilen der Detektoreinheit miteinander kombiniert werden.
